# EUROPEAN PATENT SPECIFICATION

(11) **EP 1 283 045 B1**
(45) Date of publication and mention of the grant of the patent: **28.05.2008**
(21) Application number: 02255491.9
(22) Date of filing: 06.08.2002
(51) Int. Cl.: A61K 31/736, A23L 1/308, A61P 11/06, A61P 17/00, A61P 37/08

(54) **Drug and food containing glucomannan for inhibiting IgE antibody**
Glucomannan enthaltendes Arzneimittel und Nahrungsmittel zur Hemmung von IgE-Antikörper
Médicament et aliment à base de glucomannane capable d'inhiber l'anticorp IgE

(30) Priority: 06.08.2001 JP 2001237993
(43) Date of publication of application: 12.02.2003
(73) Proprietor: Nishikawa Rubber Co., Ltd., Hiroshima-shi, Hiroshima-ken 733-8510 (JP); Shimizu Chemical Corporation, 3622, Kihara-cho Mihara-shi Hiroshima 729-0321 (JP)
(72) Inventor: Onishi, Nobukazu, Nishikawa Rubber Co. Ltd, Nishi-ku, Hiroshima-shi, Hiroshima (JP); Hashimoto, Kunihiko, Nishikawa Rubber Co. Ltd, Nishi-ku, Hiroshima-shi, Hiroshima (JP); Shimizu, Hisao, Shimizu Chemical Corporation, Mihara-shi, Hiroshima (JP)
(74) Representative: Jones, Helen M.M.

(56) References cited:
- WO-A-00/51580
- WO-A-01/33975
- US-A- 5 173 321
- US-A- 5 536 521
- DATABASE WPI Section Ch, Week 199751 Derwent Publications Ltd., London, GB; Class A11, AN 1997-550747 XP002224246 & CN 1 129 706 A (UNIV HUANAN AGRIC), 28 August 1996 (1996-08-28)
- DATABASE WPI Section Ch, Week 198229 Derwent Publications Ltd., London, GB; Class D21, AN 1982-60693E XP002224247 & JP 57 095905 A (KYOEI KAGAKU KOGYO KK), 15 June 1982 (1982-06-15)
- TANABE S ET AL: "Isolation and characterization of a novel polysaccharide as a possible allergen occurring in wheat flour." BIOSCIENCE, BIOTECHNOLOGY, AND BIOCHEMISTRY, (2000 AUG) 64 (8) 1675-80. , XP001109161
- WERLEY M S ET AL: "Respiratory sensitization to konjac flour in guinea pigs." TOXICOLOGY, (1997 DEC 26) 124 (2) 115-24. , XP002224244
- DATABASE CA [Online] CHEMICAL ABSTRACTS SERVICE, COLUMBUS, OHIO, US; TANABE, SOICHI: "Identification of wheat allergens" retrieved from STN Database accession no. 136:339612 XP002224245 & INTERNET SYMPOSIUM ON FOOD ALLERGENS [ONLINE COMPUTER FILE] (2001), 3(4), 163-170 ,
- DATABASE WPI Section Ch, Week 200268 Derwent Publications Ltd., London, GB; Class B04, AN 2002-171749 XP002224248 & JP 2002 226353 A (UNITIKA LTD), 14 August 2002 (2002-08-14)
- NOBUKAZU ONISHI ET AL: "A new immunomodulatory function of low-viscous konjac glucomannan with a small partical size: its oral intake suppresses spntaneously occurring dermatitis in NC/Nga mice" vol. 136, 2005, pages 258-265,

## Description

### FIELD OF THE INVENTION

This invention relates to IgE antibody inhibitors and foods, particularly, it relates to an IgE antibody inhibitor and food from which type I allergic disease onset prevention and the like actions can be expected.

### BACKGROUND OF THE INVENTION

Accompanied by the changes in dietary life, residential environment and the like, morbidity rate and mortality rate of allergic diseases are showing a worldwide increasing tendency for the past 10 years. According to a private investigation ("The Present Situation and Future Prospect on New Drug Development" '91 edition, Seed Planning), one in every three people in Japan are currently showing atopic dermatitis, bronchial asthma, allergic rhinitis and the like symptoms of typical type I allergic diseases. These data are also supported by the Investigation on Health and Welfare Trends, the Ministry of Health and Welfare, (1991). Though allergic diseases are rarely concerned in mortal danger directly, they suddenly appear in very younger generations and become chronic because spontaneous cure at early stage can hardly be expected. Accordingly, not only the burden to the patients and their families as a matter of course, but they also exert great influences on social activities for a prolonged period of time.

It is considered that the type I allergic diseases are sensitized and induced by the following mechanism. Firstly, when indoor dust, mite, pollens, fungi and the like antigens are inhaled, B cells release IgE antibodies by the action of CD4 positive T cells which produce Th2 type cytokine. Sensitization is established by further binding to receptors on mast cells at the Fc fragment of IgE antibodies. Next, histamine, leukotriene and the like chemical mediators are released by cross-linking of the Fab fragment of the IgE antibodies on the surface of mast cells by the reinvaded antigens. These substances cause inflammation of tissues, acceleration of vascular permeability, contraction of smooth muscles, acceleration of mucus secretion and the like and thereby induce morbid states of allergic diseases.

The most effective method for treating allergic diseases is to avoid contact with antigens. However, the patients sensitized and induced by antigens which are released and present everywhere in the residential environment have to depend on a temporary resolving means using a symptomatic therapy drug such as an antihistaminic which shows side effects. Onset of the diseases is repeated unless continuing internal use or application of drugs, and there is a fear of worsening the symptoms by rebound when their use is suspended. Because of this, such patients are forced to have great burdens economically and physically.

US-A-5,536,521 discloses a rapidly hydratable konjak flour having particular viscosity characteristics. Said Konjak flour is used in the food industry.

US-A-5,173,321 reports the preparation of flavoured Konnyaku, a refined glucomannan, and food products containing it.

A process for producing refined Konjak starch containing high levels of glucomannan, high viscosity and water solubility is disclosed in CA-A-1,129,706. The use of cosmetic compositions comprising refined konjak powder, glucomannan and their aqueous solution is described in JP-A-57-095905. Compositions containing sphingoglycolipids derived from konjak flour used to treat atopic and allergic dermatitis are disclosed in WO-A-0205662 (which was not published at the priority date and is thus only relevant to the novelty of the present invention).

WO-A-0051580 details a glycophosphopeptical, a glucomannan from Candida utilis, which is used in the treatment and/or prophylaxis of diseases caused by type I hypersensitivity reactions, including asthma allergic rhinitis and atopic dermatitis.

The use of non-digestible hydrolysed glucomannans in nutritional compositions is proposed in WO-A-0133975 to reduce the uptake of allergens, such as IgE through the intestinal wall, thus allowing the prevention and/or prophylaxis of allergic reactions.

However, some glucomannans appear to elicit an induction of allergic reactions rather than an inhibition thereof. Wheat polysaccharides comprising mannan and glucan and having a H-NMR spectrum similar to konjacglucomannan are found to act as allergens in Bioscience, Biotechnology and Biochemistry 64(8) 1675-80 (2000). It is further reported in Toxicology 124(2), 115-24 (1997) that food grade konjac flour is responsible for allergic asthma. In Symposium on Food Allergies 3(4), 163-170 (2001), mannoglucan is identified as a potential wheat allergen responsible, together with wheat proteins, for baker's asthma and atopic dermatitis.

### SUMMARY OF THE INVENTION

Taking the aforementioned problem into consideration, an object of the invention is to provide an IgE antibody inhibitor and food, which can control IgE antibody titer in vivo, prevent onset of atopic dermatitis, bronchial asthma, allergic rhinitis and the like allergic diseases, and can treat an improve morbid states even when these diseases are induced, and which are safe and easy to intake.

The "living body" addressed herein includes those of warm-blooded animals, preferably mammals, more preferably human.

To achieve the above-described objects, the present inventors have conducted extensive studies in order to develop a drug or food having a function to improve morbid states of allergic diseases by inhibiting production of IgE antibodies. As a result, the inventors found for the first time that glucomannan has a markedly high IgE antibody inhibitory capacity and a function to prevent allergic diseases. The present invention has been accomplished based on this finding.

That is, the above-described objects of the invention have been achieved by the following:
1) Use of glucomannan powder that is in the form of refined konjak flow, is easily soluble in water and has an average particle diameter of 100 µm or less, a weight average molecular weight of 1,000,000 or more and a period of time until its 1% aqueous solution reaches the viscosity peak at room temperature being within 30 minutes, in the manufacture of a composition for oral administration to a human or animal for inhibiting, preventing or treating allergic diseases.
2) Use of a composition described in the above item (1) wherein the glucomannan has a dietary fiber content of 95% or more.
3) Use of a composition described in the above item (1), in which the composition has a form of powder, capsule, tablet, pill or granule.
4) Use of a composition described in any one of the items (1) to (3), wherein the product is a food.

### BRIEF DESCRIPTION OF DRAWINGS

Figure 1 is a graph showing a result of Example 1.
Figure 2 shows photographs showing skin disease conditions of atopic dermatitis spontaneous on set mode mice.

### BRIEF DESCRIPTION OF THE INVENION

In the glucomannan of present invention is the main component of a composition having antibody inhibitor properties. Glucomannans have a long period of actually used results as a food material and a food additive particularly in Japan and also have high safety. Accordingly, its continuous internal use is possible.

Refined *konjak* flour of easy availabity. The refined *konjak* flour to be used in the invention is described in detail in "Science of Konjak (established in 1993)" edited by Satoshi Okimasu. The terminology "konjak" [kon-nyaku] as used herein means *Amorphophallus Konjac,* which has hitherto been eaten as food, especially in Japan, and which may be called as "devil's tongue".

As the aforementioned glucomannan, it is desirable that its dietary fiber content is 95% or more. The method for controlling the dietary fiber content within the above range is not particularly limited, but it is desirable to obtain purified glucomannan by purifying the aforementioned refined konjak flour by an ethanol precipitation method.

Also, it is required that the aforementioned glucomannan is easily soluble in water. Though the method for making glucomannan into easily water-soluble property is not particularly limited, a pulverization treatment is desirable from the viewpoint of easy workability.

It is required that the glucomannan made into easily water-soluble state by the pulverization treatment as described above has a weight average molecular weight of 1,000,000 or more and a period of time until its 1% aqueous solution reaches the viscosity peak at room temperature within 30 minutes.

The composition which is an IgE antibody inhibitor may be embodied in any form of powder, gelatin capsule and the like capsules, tablets, pills or granules. Also, it may be used together with a filler or may contain other auxiliary component so long as it does not spoil functions of the IgE antibody inhibitor. Any substance which is harmless to human can be used as the auxiliary component which may be contained.

Intake of the glucomannan of the invention is effective generally at an oral dose of from 1 to 50 g/60 kg body weight per day.

In the case of allergic diseases caused by excess IgE antibody production, the glucomannan of the invention can be used as an allergic disease protecting agent or allergic disease preventing agent.

In addition, it may be embodied also in a form in which it is contained in general food, namely as an IgE antibody inhibitory food.

In order to obtain an IgE antibody inhibitory food, the purified glucomannan of the invention may be blended in response to the property of respective food, for example in a powdery form with a biscuit-like food. Its minimum concentration in food effective in exerting the effects of the invention is 1% by weight or more in terms of the amount of purified glucomannan.

### EXAMPLES

The present invention will be illustrated in greater detail with reference to the following Examples,

### Example 1

### Analysis of the amount of IgE antibody in sera:

### <Test Methods>

As the animal to be tested, a spontaneously induced atopic dermatitis model animal NC/nga mouse (hereinafter referred to as "NC mouse") [Matsuda H et al.; Int. Immunol., 9, 461 (1997)] was used, and males of 4 weeks of age were purchased from Japan SLC. Using 5 animals of the NC mouse as one group, a basal feed administered group, a test feed 1 administered group, a test feed 2 administered group and a test feed 3 administered group were set, and each group was allowed to feed on the basal feed, test feed 1, test feed 2 and test feed 3 freely for 8 weeks.

Feeding MF (solid feed) manufactured by Oriental Yeast, Co., Ltd. was used as the basal feed. Respective test feed was used by adding 5% by weight of each of the following additives to the basal feed.

The additives to be added to respective feed are shown below.

Test feed 1: refined *konjak* flour (mfd. by Shimizu Kagaku)

Test feed 2: purified high purity glucomannan having the dietary fiber content of 99% or more (mfd. by Shimizu Chemical, trade name "PROPAL A")

Test feed 3: finely pulverized purified glucomannan made into easily water-soluble state by applying a pulverization treatment (mfd. by Shimizu Chemical)

Also, data on these additives are shown in Table 1 below.

**Table 1**

| Measured items | Refined konjak flour | Purified glucomannan | finely pulverized purified glucomannan |
|---|---|---|---|
| Average particle diameter (µm) | 274 | 301 | 99 |
| Viscosity peak reaching time (hr) | 4.0 | 7.0 | 0.5 |
| Viscosity (cpa) | 56,200 | 123,700 | 35,100 |
| Weight average molecular weight | 0.98 x 10⁶ | 1.92 x 10⁶ | 1.90 x 10⁶ |
| Dietary fiber content (%) | 75 | 98.5 | 96.8 |

| | | | |
|---|---|---|---|
| (Note) The viscosity peak reaching time and the viscosity were measured by dissolving each sample in an aqueous solution of 25°C to a concentration of 1$. Also, the viscosity indicates a value after the viscosity peak reaching time. | | | |

Blood samples were collected from eye veins of all NC mice at an interval of 2 weeks. The blood samples were centrifuged at 1,700 rpm for 10 minutes to obtain sera. Total IgE antibody titers in the thus obtained sera were analyzed by the sandwich ELISA method.

### <Test Results>

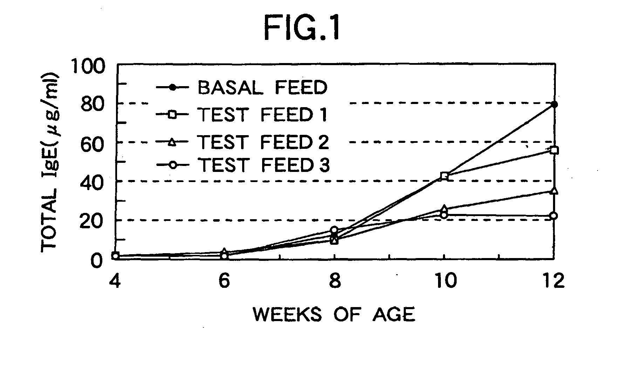

Results of the above test are shown in Fig. 1. The axes of ordinate and abscissa in the drawing respectively show the total IgE content and weeks of age of NC mice.

As shown in Fig. 1, significant increase in the serum IgE antibody content was confirmed until 12 weeks of age in the basal feed administered group. On the other hand, IgE antibody inhibitory action was observed in the groups to which the glucomannan of the invention was administered (test feed 1 administered group, test feed 2 administered group and test feed 3 administered group) when compared with the basal feed administered group. Particularly, the effect was significant in the test feed 2 and 3 administered groups in and after 8 weeks of age. At the time of 12 weeks of age, the IgE antibody production in the test feed 2 administered group was inhibited to about 50% of the basal feed administered group, and about 30% in the test feed 3 administered group.

### Example 2

### Observation of changes in morbid state in allergic disease model mice:

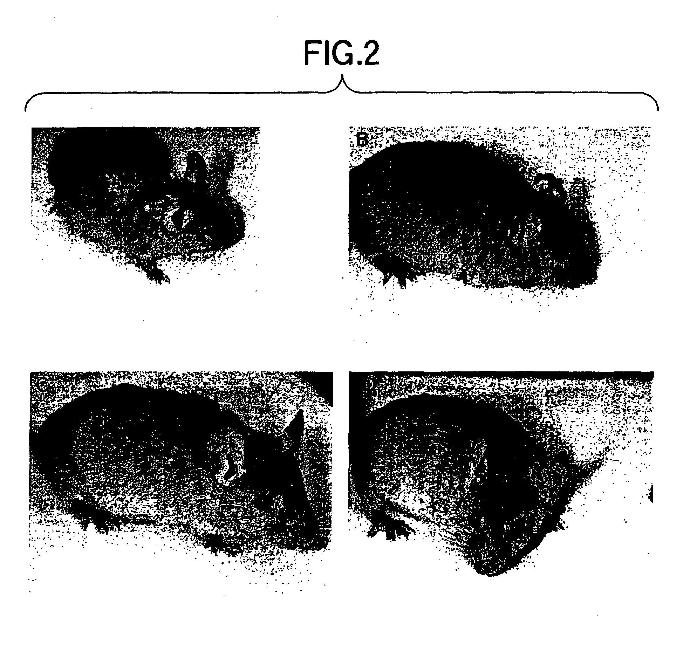

Changes in the morbid state of skin conditions of each of the test animal groups used in Example 1 were observed with the naked eye, with the appearance of the skin disease conditions at the time of 12 weeks of age shown in Fig. 2. The photographs A, B, C and D shown in Fig. 2 are the basal feed administered group, the test feed 1 administered group, the test feed 2 administered group and the test feed 3 administered group, respectively.

In the basal feed administered group and the test feed 1 administered group, loss of hair and bleeding were observed on the cranial region, cervical region and auricle region starting around 9 weeks of age. In addition, deletion of auricle and onset of dermatitis caused by itching behavior were also observed. However, such changes in morbid state were not observed in the test feed 2 administered group and test feed 3 administered group.

As described in the above, the IgE antibody inhibitor and food of the invention can inhibit IgE antibody production in the living body and prevent allergic diseases, because they contain the glucomannan of the invention. In addition, these effects become more significant by the use of purified glucomannan having a dietary fiber content of 95% or more.

## Claims

1. Use of glucomannan powder that is in the form of refined konjak flour, is easily soluble in water and has an average particle diameter of 100 µm or less, a weight average molecular weight of 1,000,000 or more and a period of time until its 1% aqueous solution reaches the viscosity peak at room temperature being within 30 minutes in the manufacture of a composition for oral administration to human or animal for inhibiting, preventing or treating allergic disease.

2. Use according to claim 1, wherein the glucomannan powder has a dietary fiber content of 95% or more.

3. Use according to claim 1 in which the composition has a form of powder, capsule, tablet, pill or granule.

4. Use according to any preceding claim in which the composition is a food.

5. Use according to claim 1 in which the disease is atopic dermatitis, bronchial asthma or allergic rhinitis.

## Patentansprüche

1. Verwendung von Glucomannanpulver, welches in Form von raffiniertem Konjakmehl vorliegt, in Wasser leicht löslich ist und einen mittleren Teilchendurchmesser von 100 µm oder weniger, ein gewichtsmittleres Molekulargewicht von 1000000 oder mehr und einen Zeitraum, bis seine 1 %ige wässrige Lösung das Viskositätsmaximum bei Raumtemperatur erreicht, welcher innerhalb von 30 Minuten liegt, aufweist, bei der Herstellung einer Zusammensetzung zur oralen Verabreichung an Menschen oder Tiere zum Hemmen, Verhüten oder Behandeln einer allergischen Erkrankung.

2. Verwendung nach Anspruch 1, wobei das Glucomannanpulver einen Ballaststoffgehalt von 95 % oder mehr aufweist.

3. Verwendung nach Anspruch 1, wobei die Zusammensetzung in Form eines Pulvers, einer Kapsel, einer Tablette, einer Pille oder eines Körnchens bzw. Granulats vorliegt.

4. Verwendung nach einem vorangehenden Anspruch, wobei die Zusammensetzung ein Nahrungsmittel ist.

5. Verwendung nach Anspruch 1, wobei es sich bei der Erkrankung um atopische Dermatitis, Bronchialasthma oder allergische Rhinitis handelt.

## Revendications

1. Utilisation d'une poudre de glucomannane qui est sous la forme d'une farine de konjac raffinée, est facilement soluble dans l'eau et a un diamètre de particule moyen de 100 µm ou moins, une .masse moléculaire moyenne en poids de 1 000 000 ou plus et une période de temps avant que sa solution aqueuse à 1 % n'atteigne le pic de viscosité à température ambiante de moins de 30 minutes, dans la fabrication d'une composition pour une administration par voie orale à l'homme ou l'animal destinée à inhiber, prévenir ou traiter une maladie allergique.

2. Utilisation selon la revendication 1, dans laquelle la poudre de glucomannane a une teneur de fibres diététiques de 95 % ou plus.

3. Utilisation selon la revendication 1, dans laquelle la composition est sous la forme d'une poudre, d'une capsule, d'une pilule ou d'un granule.

4. Utilisation selon l'une quelconque des revendications précédentes, dans laquelle la composition est un aliment.

5. Utilisation selon la revendication 1, dans laquelle la maladie est l'eczéma constitutionnel, l'asthme bronchique ou la rhinite allergique.
